(19) **Europäisches Patentamt**
**European Patent Office**
**Office européen des brevets**

(11) Publication number: **0 612 516 B1**

(12) **EUROPEAN PATENT SPECIFICATION**

(45) Date of publication of patent specification: **20.12.95**

(51) Int. Cl.⁶: **A61K 7/42**, A61K 7/48, A61K 7/00, C09C 1/00, C09C 1/36

(21) Application number: **93118804.9**

(22) Date of filing: **23.11.93**

The file contains technical information submitted after the application was filed and not included in this specification

(54) **Skin cosmetics**

(30) Priority: **02.02.93 JP 36225/93**

(43) Date of publication of application:
**31.08.94 Bulletin 94/35**

(45) Publication of the grant of the patent:
**20.12.95 Bulletin 95/51**

(84) Designated Contracting States:
**DE FR GB**

(56) References cited:
**EP-A- 0 192 485**
**EP-A- 0 359 909**
**EP-A- 0 526 712**
**DE-A- 4 038 258**
**DE-A- 4 137 764**

**PATENT ABSTRACTS OF JAPAN vol. 13, no. 273 (C-609)(3621) & JP-A-01 068 313 (HITACHI METALS LTD)**

**S.T.N. File Supplier, KARLSRUHE, DE File Chemical Abstracts, vol 101, n 116589 * abstract ***

(73) Proprietor: **Degussa Aktiengesellschaft**
**Weissfrauenstrasse 9**
**D-60311 Frankturt (DE)**

Proprietor: **SEVEN CHEMICAL CO., LTD.**
**1-12-1, Taiheiji,**
**Kashiwara**
**Osaka (JP)**

(72) Inventor: **Shono, Yoshio**
**Kasuga 98-53, Taishi-cho**
**Minamikawachi-gun,**
**Osaka Prefecture (JP)**
Inventor: **Fukuma, Tadashi**
**Hachikawa 149-10, Taima-cho**
**Kitakatsuragi-gun,**
**Nara Prefecture (JP)**

(74) Representative: **Weber, Wolfgang**
**Degussa AG**
**Fachbereich Patente**
**Postfach 1345**
**D-63403 Hanau (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

DATABASE WPI Week 9034, Derwent Publications Ltd., London, GB; AN 90-256407 & JP-A-2 178 219 (SHOKUBAI KASEI KOGYO) 11 July 1990

PATENT ABSTRACTS OF JAPAN vol. 11, no. 115 (C-415)(2562) & JP-A-61 257 910 (POLA CHEM IND INC)

PATENT ABSTRACTS OF JAPAN vol. 14, no. 314 (C-737)(4257) & JP-A-02 108 612 (SHIT-ZEN K. K.)

## Description

This invention relates to skin cosmetics such as, for example, solidified cosmetics, emulsified cosmetics and sunscreening cosmetics, which contain new fine composite oxides.

As well known, titanium oxide (with an average particle size of 0.1 $\mu$m or less) is effective for shielding UV-ray and thus capable of providing cosmetics with a sun-screening effect (JP-PS 49-000450 and JP-PS 47-42502).

However, it is not dispersed homogeneously in cosmetics due to strong aggregation caused by its fineness. As a result of this, cosmetics containing it show poor extensibility and some stiffness (not smooth but rough feeling with poor adhesion) on the skin. Besides, such cosmetics emphasize whiteness unnaturally and have a strong tendency to show dull tone (dark and unpleasant color) after make-up. For these reasons, it is not possible to add fine titanium oxide to a sufficient extent.

It is also known to formulate cosmetics with iron oxide. They show different dispersibility in the cosmetics in accordance with their type. Especially, red iron oxide and black iron oxide are inferior in dispersibility. Therefore, cosmetics containing these oxides have shown deficiencies like the appearance of coarse pigment particles or the destruction of other powders in cosmetics after a long mixing process during production.

The subject of the invention are skin cosmetics containing fine composite oxides composed of titanium oxide obtained by flame hydrolysis and iron oxide obtained by flame hydrolysis with an average particle size of 5 to 100 nm

The inventors examined fine oxide particles for cosmetics considering such deficiencies as mentiond above and have persued the matter of this invention by finding the fact that fine composite oxides consisting of titanium oxide and iron oxide obtained by flame hydrolysis show, when added to cosmetics, a remarkably good capability of improving its UV-ray shielding effect in comparison with conventional fine tianium oxide particles to achieve cosmetic products ensuring complexionlike coloring without emphasizing whiteness unnaturally and causing a dull tone after make-up.

The purpose of this invention is to provide skin cosmetics with excellent UV-ray shielding effect which show superior extensibility and adhesiveness when applied to skin and ensure a transparent complexion, free from dull tone, and without causing unnatural emphasis of whiteness like titanium oxide.

For achieving the purpose as mentioned above, skin cosmetics based on this invention contain fine composite oxides with an average particle size between 5 and 100 nm consisting of titanium oxide and iron oxide obtained by flame hydrolysis. The composite oxides used in this invention are obtained by the so-called flame hydrolysis method.

For example, it is known to produce fine particles of titanium, silicon or aluminium oxide in a hydrolysis reaction of the vapour of titanium, silicon or aluminium halide in the flame of a burner of a gas such as hydrogen, for example, which forms water during combustion in oxygen (or air) (see Tokkyo Kokoku = Patent Publication SHO-36-3359 and SHO-47-46274, for example). The composite oxides used in this invention can easily be obtained by applying such method. For example, if the vapour of titanium tetrachloride as the main material mixed with the vapour of iron chloride ($FeCl_3$) at any ratio as required is fed into a mixture of hydrogen and air (or oxygen) and subjected to combustion by means of a burner and reaction in its flame, fine composite oxides of titanium and iron can be obtained.

The titanium oxide powder which contains iron oxide can be the same as described in the German patent application P 43 02 896.2 filed on February 2nd, 1993. This titanium oxide powder is characterized in that it consists of a fumed, prefered produced by a flame hydrolysis iron oxide titanium oxide mixed oxide showing a BET-surface of 10 to 150 $m^2$/g, containing 0,5 to 50 % by weight of iron oxide, refering to the total amount of the mixed oxides.

Preferably the iron oxid titanium oxid mixed oxide is characterized in the following datas:

| | |
|---|---|
| Content of iron oxide | 0,5 to 50 % by weight |
| BET-surface area | 10 to 150 $m^2$/g |
| Average particle size | 5 to 100 nm |
| Tamped density | 100 to 300 g/l |
| Ignition loss (2h at 1000 °C) | 0,5 to 5 % by weight |
| Chloride content | < 1 ..% by weight |

This iron oxid titanium oxide mixed oxide can be produced by the following methode:
Iron-III-chlorid, which is free of water, is vaporized, mixed with nitrogen gas, and in a known burner then

3

mixed with hydrogen air and titanium chloride in a proportion which corresponds to the proportion of the iron oxide titanium oxide mixed oxide, and then burned in a reaction chamber.

The content of iron oxide in titania can be controlled as desired by changing the feed rate of iron halide as its raw material.

The said composite oxides does not consist of a mere mixture of titanium oxide and iron oxide. But, individual fine particles of titanium oxide as the main part are chemically bonded with iron oxide at a certain ratio to form such composite oxides.

The average size of fine particles is very small within a range between 5 and 100 nm. The particle size and composition of oxides thus obtained can be adjusted by changing the feed rate of combustion gas mixtures. Fine particles consisting of titanium oxide as the main material and iron oxide between 0.05 to 50 % by weight have proved to represent those composite oxides which show excellent effects as the additives for various skin cosmetics because of their superior UV-ray shielding effect, excellent dispersibility in liquids and powders as well as good extensibility on skin without causing any dull tone.

It is preferable as a rule to add such fine composite oxides to the basic material of skin cosmetics withtin a range from 0.05 to 10 % by weight.

Example 1

FeCl$_3$ and TiCl$_4$ are vaporized separately at a temperature of 400 °C for FeCl$_3$ and 200 °C for the TiCl$_4$. The vapours of the chlorides were mixed with nitrogen and passed to the mixing chamber of a burner. There they are mixed with hydrogen and dried air and/oder oxygen and burned. The resulting products are cooled down to ca. 100 °C and seperated from the reaction gases by a filter. The chloride is removed hy handling the products with wet air at a temperature of 500 to 700 °C. Table 1 shows the conditions of the reaction and the quality of the resulting products of different mixed oxides according to the invention.

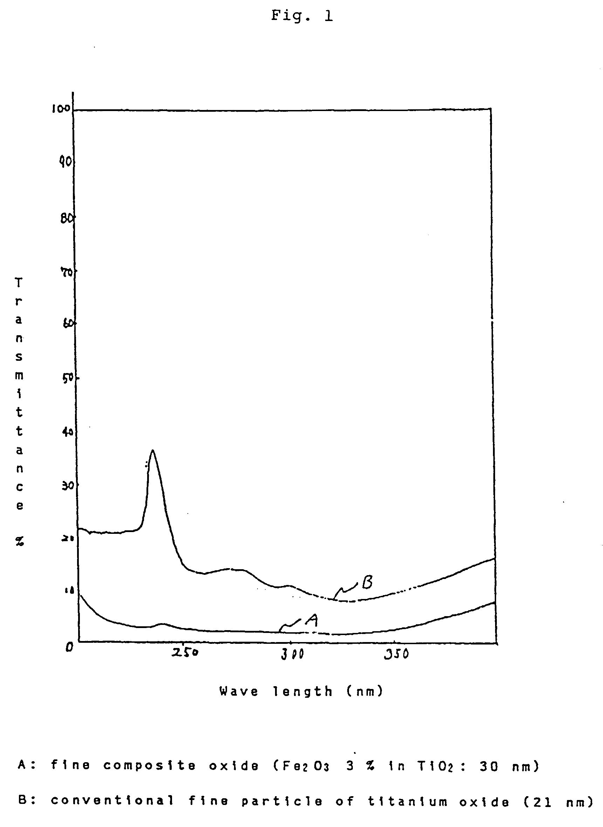

Fig. 1 shows the relation between transmittance and transmission wave length of UV-ray for fine composite oxides (average particle size: 30 nm) consisting of titanium oxide and iron oxide (3 %) and for conventional fine particles of titanium oxide (average particle size: 21 nm). For this measurement, the said both particles were added to a mixture of isopropyl myristate and vaseline (3 : 7) at a ratio of 0.25 %, mixed well and put between 2 quartz plates in a thickness of 0.2 mm. Spectro-photometer UV-201A manufactured by Shimazu Corporation was used for this measurement.

The axis of ordinates shows the transmittance in %, while that of abscissas shows the wave length in nm. Curve A represents the result with the fine composite oxide as stated above, while curve B indicates that with the said conventional fine particles of titanium oxide.

As demonstrated by this comparison, the sample containing the fine composite oxide based on this invention shows remarkably lower transmittance of UV-ray than that containing conventional fine particles of titanium oxide at a wave length from 250 to 350 nm. As this range of UV radiation causes sunburn most strongly, the fine composite oxide provides excellent protection by shielding UV-ray in said range.

Table 1

Iron oxide titanium oxid mixes oxide

| Nr. | TiCl4 (g/h) | FeCl3 (g/h) | H2 (l/h) | Air (l/h) | $Fe_2O_3$ (%) | BET surface $(m_2/g)$ | Tamped density (g/l) | Ignition loss (%) | Chloride content (%) |
|---|---|---|---|---|---|---|---|---|---|
| 1 | 1717 | 14 | 525 | 3079 | 0,9 | 51 | 185 | 2,1 | 0,25 |
| 2 | 1687 | 47 | 525 | 3079 | 3,1 | 47 | 190 | 2,1 | 0,44 |
| 3 | 1613 | 107 | 525 | 3079 | 7,2 | 46 | 185 | 1,7 | 0,36 |
| 4 | 1577 | 161 | 525 | 3079 | 10,7 | 47 | 180 | 1,9 | 0,35 |
| 5 | 1424 | 326 | 525 | 3079 | 21,1 | 49 | 180 | 1,8 | 0,25 |

Example 2

A sun-screen creme was prepared by the following formulation and production method.

| | | | wt % |
|---|---|---|---|
| 1. | Fine composite oxides | | 5.0 |
| 2. | Oleophilic monostearic glyceride | | 3.0 |
| 3. | Cetyl alcohol | | 3.0 |
| 4. | Vaseline | | 3.0 |
| 5. | Squalene | | 7.5 |
| 6. | Isopropyl myristante | | 7.5 |
| 7. | Methoxy octyl cinnamate | | 5.0 |
| 8. | Polyoxy ethylene sorbitan monolaurate (20 E.O.) | | 2.0 |
| 9. | 1.3-butylene glycol | | 3.0 |
| 10. | Glycerin | | 2.0 |
| 11. | Preservative | | proper q'ty |
| 12. | Purified water | | 59.0 |
| 13. | Aromatics | | proper q'ty |
| | | | Total 100.0 wt% |

Production method:

Materials 9 through 13 were dissolved while heating and held at 75 °C. Then, material 1 was added and dispersed. This mixture was added to materials 2 through 8 having been dissolved and dispersed in advance while heating at 75 °C. After the mixture having been cooled down to 50 °C while stirring, material 13 was added and the mixture was further cooled down to 30 °C while stirring to obtain a sun-screen creme.

Example 3 (comparison)

A sun-screen creme was obtained under similar conditions to those for example 2 except for the use of 4.9 % conventional fine particles of titanium oxide and 0.1 % red iron oxide instead of the fine composite oxides used in the example 2.

Example 4

A sun-screen creme was prepared by the following formulation and production method.

|  |  |  | wt % |
|---|---|---|---|
| 1. | Fine coomposite oxides | | 5.0 |
| 2. | Titanium oxide | | 5.0 |
| 3. | Talc | | 20.0 |
| 4. | Sericite | | 51.2 |
| 5. | Yellow ferric oxide | | 1.3 |
| 6. | Red oxide of iron | | 0.51 |
| 7. | Black iron oxide | | 0.19 |
| 8. | Nylon powder | | 5.0 |
| 9. | Methylhydrogen polysiloxyne | | 1.8 |
| 10. | Methyl polysiloxane | | 1.5 |
| 11. | Glyceryl di-iso-stearate | | 2.0 |
| 12. | Lanoline refined by adsorption | | 1.5 |
| 13. | Octyl dodecanol | | 1.0 |
| 14. | Squalene | | 4.0 |
| 15. | Preservative | | proper q'ty |
| 16. | Aromatics | | proper q'ty |
|  |  | | Total 100.0 % |

Production method:

Materials 1 through 8 were mixed first in a Henschel mixer, to which material 9 was then added. After that, materials 10 and 16 as well as materials 11 through 15 dissolved by heating were added to said mixture and blended together. The mixture thus obtained was put on a metal dish and formed under pressure into a foundation for both dry and wet use.

Example 5 (comparison)

A foundation was obtained under similar conditions to those for example 4 except for the use of conventional fine particles of titanium oxide instead of the fine composite oxide used in the example 4.

Samples based on the examples 2 and 4 as well as on the examples 3 and 5 for comparison above were put to practical use by 15 women for judging their coloring effect, extensibility and stay on skin, appearance of color (unnatural emphasis of whiteness), etc. on the basis of the following criteria for evaluation. The results are shown in Table 2.

As described above, the skin cosmetics based on this invention do not only exhibit an excellent protective effect against UV-ray due to superior UV-ray shielding performance of the fine composite oxides contained in them, but also have beautiful appearance of color as a result of good dispersibility of such fine composite oxides in various basic materials for skin cosmetics. Furthermore, they provide excellent make-up effects on the basis of good extensibility and stay on skin with no rough but smooth feeling without any unnatural emphasis of whiteness immediately after application or any dull tone of the skin during the time of wearing of the make-up as the very phenomena to be avoided in make-up.

Table 2

|  | Exemple 2 | Exemple 3 | Exemple 4 | Exemple 5 |
|---|---|---|---|---|
| Appearance of color of product | 4 | 2 | 4 | 2 |
| Extensibility on skin | 4 | 2 | 5 | 3 |
| Adhesiveness to skin | 4 | 3 | 4 | 3 |
| Complexion development | 5 | 2 | 5 | 2 |
| Feeling at use | 5 | 3 | 4 | 3 |
| Evaluation criteria:<br>1: bad; 2: rather bad; 3: normal; 4: good; 5: very good | | | | |

## Claims

1. Skin cosmetics containing fine composite oxides composed of titanium oxide obtained by flame hydrolysis and iron oxide obtained by flame hydrolysis with an average particle size of 5 to 100 nm.

## Patentansprüche

1. Feine Verbundoxide enthaltende Hautkosmetika, wobei die besagten Verbundoxide aus durch Flammenhydrolyse gewonnenem Titanoxid und durch Flammenhydrolyse gewonnenem Eisenoxid bestehen und eine durchschnittliche Teilchengröße von 5 bis 100 nm aufweisen.

## Revendications

1. Cosmétiques pour la peau contenant des oxydes composites fins formés d'oxyde de titane obtenu par hydrolyse à la flamme et d'oxyde de fer obtenu par hydrolyse à la flamme ayant une taille moyenne de particules allant de 5 à 100 nm.

Fig. 1

A: fine composite oxide (Fe$_2$O$_3$ 3 % in TiO$_2$: 30 nm)

B: conventional fine particle of titanium oxide (21 nm)